# EUROPEAN PATENT SPECIFICATION

(11) **EP 2 413 939 B1**
(45) Date of publication and mention of the grant of the patent: **22.04.2015**
(21) Application number: 10708493.1
(22) Date of filing: 01.03.2010
(51) Int. Cl.: A61K 31/522, A61K 31/7068, A61P 9/10, A61P 39/00, A61P 39/06

(54) **PHARMACEUTICAL COMPOSITION FOR NEUROPROTECTIVE TREATMENT IN PATIENTS WITH ICTUS COMPRISING CITICOLINE AND URIC ACID**
CITICOLIN UND HARNSÄURE ENTHALTENDE PHARMAZEUTISCHE ZUSAMMENSETZUNG ZUR NEUROPROTEKTIVEN BEHANDLUNG VON PATIENTEN MIT IKTUS
COMPOSITION PHARMACEUTIQUE POUR LE TRAITEMENT NEUROPROTECTEUR CHEZ DES PATIENTS AYANT SUBI UN ACCIDENT CÉRÉBROVASCULAIRE

(30) Priority: 30.03.2009 ES 200900856
(43) Date of publication of application: 08.02.2012
(73) Proprietor: Hospital Clinic i Provincial de Barcelona, 08036 Barcelona (ES)
(72) Inventor: CHAMORRO SÁNCHEZ, Ángel, E-08036 Barcelona (ES)
(74) Representative: Carvajal y Urquijo, Isabel
(86) International application number: PCT/EP2010/001239
(87) International publication number: WO 2010/112113

(56) References cited:
- WO-A1-96/27380
- CHAMORRO ANGEL ET AL: "Uric acid administration for neuroprotection in patients with acute brain ischemia." MEDICAL HYPOTHESES 2004 LNKD- PUBMED:14962621, vol. 62, no. 2, 2004, pages 173-176, XP002585039 ISSN: 0306-9877
- AMARO SERGIO ET AL: "A pilot study of dual treatment with recombinant tissue plasminogen activator and uric acid in acute ischemic stroke." STROKE; A JOURNAL OF CEREBRAL CIRCULATION JUL 2007 LNKD- PUBMED:17525395, vol. 38, no. 7, July 2007 (2007-07), pages 2173-2175, XP002585040 ISSN: 1524-4628
- ROMANOS EDUARDO ET AL: "Uric acid reduces brain damage and improves the benefits of rt-PA in a rat model of thromboembolic stroke." JOURNAL OF CEREBRAL BLOOD FLOW AND METABOLISM : OFFICIAL JOURNAL OF THE INTERNATIONAL SOCIETY OF CEREBRAL BLOOD FLOW AND METABOLISM JAN 2007 LNKD- PUBMED:16596120, vol. 27, no. 1, January 2007 (2007-01), pages 14-20, XP002585041 ISSN: 0271-678X

## Description

### Field of the invention

The present invention refers to the field of biomedicine and particularly to a new pharmaceutical composition comprising uric acid and citicoline and its use for the neuroprotective treatment of patients with ictus.

### Background of the invention

Cell death after a stroke is the result of the complex interaction of excitotoxicity, acidosis, inflammation, oxidative stress, peri infarct depolarization and apoptosis.

The term apoptosis is used as a synonym of programmed cell death (PCD); however, apoptosis was originally defined as a set of morphological changes which occur after PCD. In developing neurons, these changes include condensation and excision of chromatin and the formation of the so-called apoptotic bodies. These changes are different from the morphological changes which characterized the inflammation caused by necrosis of the cytoplasmic organelles and the breaking of the mitochondrial and cytoplasmic membrane.

A mild ischemic injury normally produces cell death through an apoptotic-like mechanism instead of through necrosis. Apoptosis activators include oxygen free radicals, ligation to death receptors, DNA damage, protease activation and ionic balance disadjustment. Several experimental studies have shown that the inhibition of apoptosis reduces the seriousness of the ischemic lesion.

The activation of caspases is a consequence of mitochondrial apoptosis. The mitochondrial dysfunction and the opening of the mitochondrial permeability transition pore can result in activation of caspases through the exit of cytochrome C towards the cytoplasm; however, there exist other different mechanisms through which mitochondrial dysfunction can contribute to ischemic neuronal death. The seriously damaged mitochondria can be incapable of maintaining the electrochemical gradient necessary for breathing and glucose oxidation. In this way, the mitochondrial dysfunction can aggravate the ischemic injury by exacerbation of the energetic failure.

The mitochondrial dysfunction also produces oxygen free radicals which injure other cell organelles and DNA. Therefore, the treatments preventing mitochondrial dysfunction could be a more powerful neuroprotective strategy than caspase inhibition.

High levels of intracellular Ca²⁺, Na⁺ and ADP make the mitochondria produce harmful levels of oxygen reactive species. Unlike other organs, the brain is particularly vulnerable to oxygen reactive species since the neurons have relatively low levels of endogenous antioxidants. The abundance of oxygen radicals causes the destruction of cell macromolecules and they participate in signaling mechanisms which produce apoptotic cell death. Ischemia activates nitric oxide synthase (NOS) and increases the generation of nitric oxide (NO), which is combined with super oxide to produce peroxynitrite, a powerful oxidation agent. The production of NO and oxidative stress are also joined by the over activation of poly(ADP-ribose)polymerase-1 (PARP-1), an enzyme for DNA repair.

After the reperfusion, there is an increase in the production of super oxide, NO and peroxynitrite. The formation of these radicals in the proximity of blood vessels plays an important role in the injury caused by reperfusion. These radicals activate the metalloproteases (MMP), which degrade collagen and laminins in the basal lamina, break the integrity in the vascular wall and increase permeability of the hematoencephalic barrier (HEB). Oxidative and nitrosilative stress also activates the recruiting and migration of neutrophils and other leucocytes to the brain vasculature, which release enzymes which additionally increase degradation in the basal lamina and vascular permeability. These events can produce a parenchymatous hemorrhage, vasogenic cerebral edema and leukocyte infiltration inside the brain.

It is known the use of citicoline for the preventive treatment of neurological and cognitive disorders associated to strokes and cranial traumatisms. Citicoline stimulates biosynthesis of the neuronal membrane structural phospholipids, as shown in studies carried out with magnetic resonance spectroscopy. Citicoline, through this action, improves the function of other membrane mechanisms, such as the functioning of ionic exchange pumps and receptors installed in it, the modulation of which is essential for correct neurotransmission. Citicoline due to its membrane stabilizing action has properties which favor re-absorption of the cerebral edema. Experimental studies have shown that citicoline inhibits activation of certain phospholipases (A1, A2, C and D), reducing the formation of free radicals, preventing the destruction of membrane systems and preserving the antioxidant defense systems, such as glutathione.

Citicoline preserves the neuronal energetic reserve, inhibits apoptosis and stimulates acetylcholine synthesis. It has also been experimentally proven that citicoline has a prophylactic neuroprotective effect in models of focal cerebral ischemia. Clinical assays have shown that citicoline significantly improve functional evolution of patients with acute ischemic stroke, coinciding with a smaller growth of the cerebral ischemic injury in neuroimaging tests. In patients with craneoencephalic traumatism, citicoline accelerates these patients' recovery and reduces the duration and intensity of the post-commotional syndrome. Citicoline improves attention and consciousness levels, and also has a positive effect on amnesia and cognitive and neurological disorders related to cerebral ischemia.

Uric acid is a potent antioxidant which blocks the reaction between superoxide anion and nitric oxide, which damages the cells when nitrosylating thyroxine residues of proteins. UA plasmatic concentration is almost 10 times higher than that of other antioxidant substances, such as vitamin C or E, and its antioxidant ability is higher. Besides, UA prevents the degradation of extra cellular superoxide dismutase, which is an essential enzyme for normal endothelial functioning. In culture of hippocampus cells, UA protects against excitotoxic damage by glutamate, stabilizing calcium homeostasis and preserving the mitochondrial function. UA has also shown the inhibition of the Fenton reaction.

In an adult rat, the administration of UA 2 hours before the occlusion of the middle cerebral artery or 1 hour after the reperfusion significantly reduces the resulting cerebral infarction, suppresses ROS accumulation and reduces lipid peroxidation. UA administration is neuroprotective in a thromboembolism model of focal cerebral ischemia of a rat and this neuroprotective effect is synergic with respect to the beneficial effect attained by rtPA.

There are studies which show the existing relation between higher levels of uric acid in blood in the moment of an ictus and a reduced neurological seriousness caused by said ictus.

As a result of important research, in the field of neurology, we have verified that the joint administration of uric acid and citicoline has a synergic effect on protection against cell death related to necrosis and apoptosis.

### Description of the invention

Thus, a first aspect of the present invention refers to a pharmaceutical composition comprising a therapeutically effective amount of uric acid or its pharmaceutically acceptable salts, and citicoline or its pharmaceutically acceptable salts for the neuroprotective treatment of patients with ictus.

In the present invention by "neuroprotective treatment" we mean the treatment which stops or slows down the sequence of biochemical and molecular events leading to cell death.

In the present invention by "patients with ictus" we mean patients who have had a stroke with an abrupt alteration of blood flow to the brain. Particularly, we refer to those who have had an ischemic ictus or cerebral infarction, thrombosis, embolism, hemorrhagic ictus, aneurism or transient ischemic attack.

In a more particular aspect, the therapeutically effective amount of uric acid or its pharmaceutically acceptable salts of the pharmaceutical composition of the present invention ranges between 1-4mg/ml.

In a more particular aspect, the therapeutically effective amount of citicoline or its pharmaceutically acceptable salts of the pharmaceutical composition of the present invention ranges between 2-4mg/ml.

In a more particular aspect, the pharmaceutical composition of the present invention comprises an aqueous vehicle. More particularly, the aqueous vehicle is physiological serum. More particularly, the physiological serum comprises 0.1% lithium carbonate and 5% mannitol.

In a more particular aspect, the pharmaceutical composition of the present invention is applied by parenteral administration, more in particular, the pharmaceutical composition of the present invention is administered intravenously.

In a second aspect, the present invention refers to the synergic combination of uric acid or its pharmaceutically acceptable salts and citicoline or its pharmaceutically acceptable salts for use in combined therapy for the neuroprotective treatment of patients with ictus.

In a more particular aspect, the combined therapy is carried out through the administration of uric acid or its pharmaceutically acceptable salts and citicoline or its pharmaceutically acceptable salts simultaneously, in a more particular aspect, the combined therapy is carried out through the sequential administration of uric acid and citicoline.

In the present invention, by "simultaneous administration" we mean that the administration of uric acid is carried out at the same time as the administration of citicoline.

In the present invention, by "sequential administration" we mean that the administration of uric acid immediately precedes the administration of citicoline or that the administration of citicoline immediately precedes the administration of uric acid.

In a more particular aspect, the administration of uric acid or its pharmaceutically acceptable salts is carried out in an amount comprised between 1-4mg/ml dissolved in physiological serum comprising 0.1% lithium carbonate and 5% mannitol. In a more particular aspect, uric acid is applied by parenteral administration, more in particular, it is administered intravenously.

In a more particular aspect, the administration of citicoline or its pharmaceutically acceptable salts is carried out in an amount comprised between 500-2000mg. In a more particular aspect, citicoline is applied by parenteral administration, more in particular, it is administered intravenously.

In a more particular aspect, the combined therapy is carried out by joint administration of uric acid and citicoline.

In the present invention, by "joint administration" we mean that the uric acid is mixed with citicoline.

In a more particular aspect, the joint administration of citicoline or its pharmaceutically acceptable salts and citicoline or its pharmaceutically acceptable salts is carried out in the form of the pharmaceutical composition of the present invention.

### Description of the drawings

Figure 1 describes the effect of uric acid and citicoline on cell death produced by oxygen and glucose deprivation (OGD). The values are the media +/- SEM (n=4). Significance: *vs. control, $ vs. OGD. (C: Control; UA: Uric acid; Cit: Citicoline; UA + Cit: Uric acid + Citicoline; OGD: oxygen and glucose deprivation.
Figure 2 shows the effect of uric acid and citicoline on the condensation of chromatin induced by OGD, (Hoechst stain). The values are the media +/- SEM (n=6).
Figure 3 shows the activity of caspase-3, 24 hours after OGD. The values are the media +/- SEM (n=2-3).

### Detailed description of the invention

Mixed cultures of neurons/glia of 18-day rat fetal embryos Sprague-Dawley were prepared as described in Petegnief, V. Saura, J. De Gregorio-Rocasolano, N., and Paul, S. M. (2001) Neuroscience 104, 223 - 234. The cells were suspended in minimum essential medium (MEM) supplemented by 10% fetal bovine serum and 100*µ*g/ml gentamicin and placed on 24-well plates previously covered with poly-L-lysine (5*µ*g/ml) (Nunc, Roskilde, Denmark) with a density of 0.6 x 106 cells/well and cultured at 37° C in an incubator with 95% atmospheric air/5% CO₂. The in vitro medium was partially changed on days 4, 7 and 10 (DIV) with MEM supplemented with B27. The cultures were used in 11/13 DIV. 11.9mM uric acid was prepared in 1.35mM lithium carbonate and 5% mannitol. Uric acid was added in a concentration of 100µM to the culture medium 60min before treatment with OGD (oxygen glucose deprivation) and it was also present during and after the OGD or normoxia in the corresponding HEPES buffering agents and the culture medium. Citicoline was added in a concentration of 100*µ*M 60 minutes before, during and after OGD or normoxia. The cultures were treated with uric acid alone, with citicoline alone or with a combination of both medicines. Brother cultures were treated with vehicle: 1.35mM lithium carbonate and mannitol at 5%.

For the treatment with oxygen and glucose deprivation (OGD), the cell cultures were incubated in a glucose-free HEPES buffering agent (10Mm HEPES, pH 7.4, 135nM NaCl, 5mM KCl, 1.8 CaCl₂, 0.62Mm MgSO₄) during 90 minutes at an hypoxia incubator with 5% CO₂/0.6% O₂. Control cultures were incubated in normoxia in the same incubator containing 5.5mM D-glucose in an incubator with 95% atmospheric air/5% CO₂. At the end of the hypoxia or normoxia episode the buffering agent was replaced with MEM + B27 without antioxidant and the cells were returned to an incubator with 95% atmospheric air/5% CO₂.

For the lactate dehydrogenase activity assay, cell death was estimated 3 hours 30 minutes after OGD and next lactate dehydrogenase (LDH) activity was measured released in the medium according to a modification of the method of (Wroblewski and LaDue, 1995). The reduction of absorbance in 0.75 NADH to 340nm was followed by a phosphate buffer (50nM, pH 7.4) in presence of 4.2mM pyruvic acid as substrate.

For Hoechst stain, the cultures were washed with PBS, they were fixed during 20 minutes in 4% paraformaldehyde at 4°C and washed with PBS. The cells were later incubated during 30 minutes with nuclear coloring agent Hoechst 33258 with a 0.1µg/ml concentration. After the washing, the cells were examined in a fluorescent microscope under UV light. A semi-quantitative analysis of the apoptotic nuclei was carried out with the analySIS software. The thresholds were selected to discriminate between brilliant coloring in the condensed apoptotic nuclei and normal nuclear coloring in healthy cells. The area corresponding to the condensed chromatin coloring (brilliant coloring) was calculated as the percentage of the total area in each field. The results were expressed as control percentage.

In order to determine the caspase-3 activity, the assay was carried out according to Valencia, A. and Moran, J. (2001) J. Neurosci. Res. 64, 284 - 297 Wrobleswski, F., and LaDue, J. S. (1955) Proc. Soc. Exp. Biol. Med. 90, 210 - 213, using 100µg of proteins and 25*µ*M Ac-DEVD-AMC as substrate. The fluorescence of AMC was monitored, generated by the split of Ac-DEVD-AMC (excitement/emission 380/460nm), every 2 minutes during 30 minutes in a Gemini XS Microplate spectrofluorometer (Molecular Probles). The enzymatic activity was calculated as a triangle of fluorescence/mg of protein/minute.

A unidirectional statistical analysis of variance (ANOVA) was carried out with the post hoc Bonferroni test to determine if the groups were significantly different. Significance: ***P<0.001, **P<0.01, *P<0.05 vs. ,control. $$ P<0.01 vs. OGD.

### Examples

### Example 1: Effect of uric acid and citicoline on death induced with OGD.

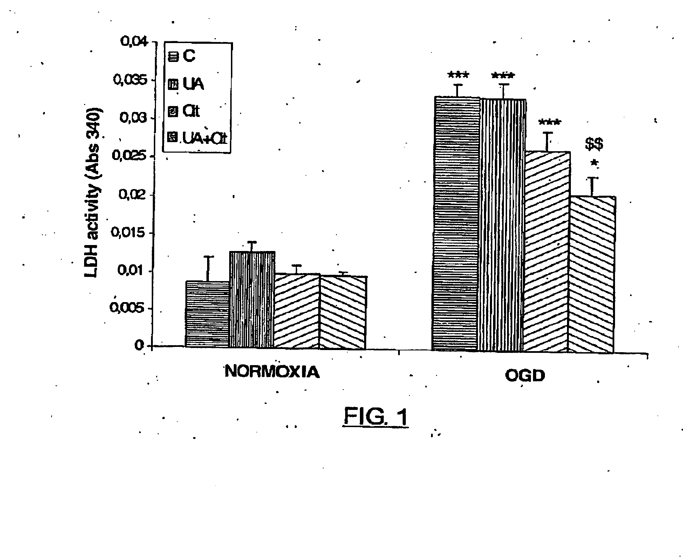

The ischemic insult induced a dramatic cell death. Actually, it the activity of lactate dehydrogenase was increased 288% (P<0.001) when compared with normoxic conditions, to the 3 hours 30 minutes of reoxigenation. As shown in **figure 1****,** the treatment with uric acid alone or with citicoline alone did not improve the cell viability in ischemic conditions. However, the combination of both treatments allowed for a significant reduction of cell death (38%, P<0.01 when compared to OGD). This showed the synergic effect of the treatment with both compounds together.

### Example 2: Joint treatment with uric acid/citicoline reduced OGD-induced condensation of chromatin.

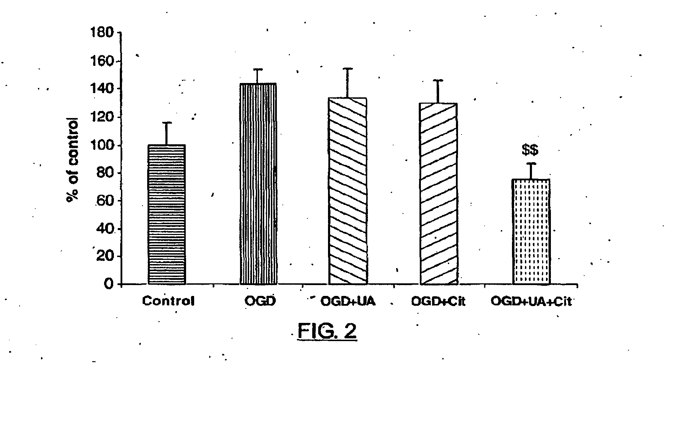

Since chromatin concentration is an indication of apoptosis, we measure this parameter 48 hours after the ischemic lesion. The treatment with OGD increased the number of apoptotic nuclei when compared to normoxia. As shown in **figure 2****,** the number of apoptotic nuclei was significantly reduced in presence of the joint treatment with uric acid + citicoline (P<0.01 vs. OGD), showing the synergic effect of both compounds when they are administered jointly.

### Example 3: Joint treatment with uric acid/citicoline inhibifed OGD-induced caspase-3 activity.

Since caspase-3 activity can contribute to apoptotic death we measure the enzymatic activity of this protease 24 hours after the OGD in our model. Preliminary data show that the ischemic insult increased caspase-3 activity in 40% and the joint treatment with uric acid/citicoline eliminated this effect, **figure 3** shows the synergic effect of both compounds.

### Example 4: Pharmaceutical composition of uric acid and citicoline

| INGREDIENTS | AMOUNT (mg) | AMOUNT (mg/ml) | EXAMPLE |
|---|---|---|---|
| Uric acid | 500-2000 | 1-4 | 1000 mg |
| Citicoline | 1000-2000 | 2-4 | 1000 mg |
| Physiological serum with 0.1 % in volume lithium and 5% mannitol | 500 ml | | 500 mg |

## Claims

1. Pharmaceutical composition comprising a therapeutically effective amount of uric acid or its pharmaceutically acceptable salts and citicoline or its pharmaceutically acceptable salts for the neuroprotective treatment in patients with ictus.

2. Pharmaceutical composition according to claim 1, **characterized in that** the therapeutically effective amount of uric acid or its pharmaceutically acceptable salts ranges between 1-4mg/ml.

3. Pharmaceutical composition according to any of the claims 1-2, **characterized in that** the therapeutically effective amount of citicoline or its pharmaceutically acceptable salts ranges between 2-4mg/ml.

4. Pharmaceutical composition according to any of the preceding claims, comprising an aqueous vehicle.

5. Pharmaceutical composition according to claim 4, **characterized in that** the aqueous vehicle is physiological serum.

6. Pharmaceutical composition according to claim 5, **characterized in that** the physiological serum comprises lithium carbonate and mannitol.

7. Pharmaceutical composition according to any of the preceding claims, **characterized in that** said pharmaceutical composition is applied by parenteral administration.

8. Pharmaceutical composition according to any of the preceding claims, **characterized in that** said pharmaceutical composition is administered intravenously.

9. Synergic combination of uric acid or its pharmaceutically acceptable salts and citicoline or its pharmaceutically acceptable salts for use in combined therapy for neuroprotective treatment of patients with ictus.

10. Synergic combination of uric acid or its pharmaceutically acceptable salts and citicoline or its pharmaceutically acceptable salts for use according to claim 9, **characterized in that** the combined therapy is carried out through the simultaneous or sequential administration of uric acid and citicoline.

11. Synergic combination of uric acid or its pharmaceutical acceptable salts and citicoline or its pharmaceutically acceptable salt for use according to any of the claims 9-10, **characterized in that** the administration of uric acid is carried out in an amount ranging between 1-4mg/ml dissolved in physiological serum with lithium carbonate and mannitol.

12. Synergic combination of uric acid or its pharmaceutically acceptable salts and citicoline or its pharmaceutically acceptable salts for use according to any of the claims 9-11, **characterized in that** the administration of citicoline is carried out in an amount ranging between 500-2000mg.

13. Synergic combination of uric acid or its pharmaceutically acceptable salts and citicoline or its pharmaceutically acceptable salts for use according to claim 9, **characterized in that** the combined therapy is carried out through the joint administration of uric acid and citicoline.

14. Synergic combination of uric acid and citicoline for use according to claim 13, **characterized in that** the joint administration of uric acid or its pharmaceutically acceptable salts and citicoline or its pharmaceutically acceptable salts is carried out in the form of a pharmaceutical composition according to any of the claims 1-8.

15. Synergic combination of uric acid or its pharmaceutically acceptable salts and citicoline or its pharmaceutically acceptable salts for use according to any of the preceding claims, **characterized in that** said synergic combination is administered via parenteral administration.

16. Synergic combination of uric acid or its pharmaceutically acceptable salts and citicoline or its pharmaceutically acceptable salt for use according to any of the preceding claims, **characterized in that** said synergic combination is administered intravenously.

## Patentansprüche

1. Pharmazeutische Zusammensetzung, die eine therapeutisch wirksame Menge von Harnsäure oder deren pharmazeutisch annehmbaren Salze und CDP-Cholin oder dessen pharmazeutisch annehmbaren Salze, für die neuroprotektive Behandlung bei Patienten mit Iktus, umfasst.

2. Pharmazeutische Zusammensetzung nach Anspruch 1, **dadurch gekennzeichnet, dass** die therapeutisch wirksame Menge von Harnsäure oder deren pharmazeutisch annehmbaren Salzen im Bereich zwischen 1-4 mg/ml liegt.

3. Pharmazeutische Zusammensetzung nach einem der Ansprüche 1-2, **dadurch gekennzeichnet, dass** die therapeutisch wirksame Menge von CDP-Cholin oder dessen pharmazeutisch annehmbaren Salzen im Bereich zwischen 2-4 mg/ml liegt.

4. Pharmazeutische Zusammensetzung nach einem der vorhergehenden Ansprüche, die einen wässrigen Trägerstoff umfasst.

5. Pharmazeutische Zusammensetzung nach Anspruch 4, **dadurch gekennzeichnet, dass** der wässrige Trägerstoff physiologisches Serum ist.

6. Pharmazeutische Zusammensetzung nach Anspruch 5, **dadurch gekennzeichnet, dass** das physiologische Serum Lithiumcarbonat und Mannitol umfasst.

7. Pharmazeutische Zusammensetzung nach einem der vorhergehenden Ansprüche, **dadurch gekennzeichnet, dass** die genannte pharmazeutische Zusammensetzung durch parenterale Verabreichung angewandt wird.

8. Pharmazeutische Zusammensetzung nach einem der vorhergehenden Ansprüche, **dadurch gekennzeichnet, dass** die genannte pharmazeutische Zusammensetzung intravenös verabreicht wird.

9. Synergetische Kombination von Harnsäure oder deren pharmazeutisch annehmbaren Salzen und CDP-Cholin oder dessen pharmazeutisch annehmbaren Salzen, zur Verwendung in der Kombinationstherapie für die neuroprotektive Behandlung von Patienten mit Iktus.

10. Synergetische Kombination von Harnsäure oder deren pharmazeutisch annehmbaren Salzen und CDP-Cholin oder dessen pharmazeutisch annehmbaren Salzen, zur Verwendung nach Anspruch 9, **dadurch gekennzeichnet, dass** die Kombinationstherapie durch die gleichzeitige oder aufeinanderfolgende Verabreichung von Harnsäure und CDP-Cholin durchgeführt wird.

11. Synergetische Kombination von Harnsäure oder deren pharmazeutisch annehmbaren Salzen und CDP-Cholin oder dessen pharmazeutisch annehmbaren Salzen, zur Verwendung nach einem der Ansprüche 9-10, **dadurch gekennzeichnet, dass** die Verabreichung von Harnsäure in einer Menge durchgeführt wird, die zwischen 1-4 mg/ml liegt, gelöst in physiologischem Serum mit Lithiumcarbonat und Mannitol.

12. Synergetische Kombination von Harnsäure oder deren pharmazeutisch annehmbaren Salzen und CDP-Cholin oder dessen pharmazeutisch annehmbaren Salzen, zur Verwendung nach einem der Ansprüche 9-11, **dadurch gekennzeichnet, dass** die Verabreichung von CDP-Cholin in einer Menge durchgeführt wird, die zwischen 500-2000 mg liegt.

13. Synergetische Kombination von Harnsäure oder deren pharmazeutisch annehmbaren Salzen und CDP-Cholin oder dessen pharmazeutisch annehmbaren Salzen, zur Verwendung nach Anspruch 9, **dadurch gekennzeichnet, dass** die Kombinationstherapie durch die gemeinsame Verabreichung von Harnsäure und CDP-Cholin durchgeführt wird.

14. Synergetische Kombination von Harnsäure und CDP-Cholin zur Verwendung nach Anspruch 13, **dadurch gekennzeichnet, dass** die gemeinsame Verabreichung von Harnsäure oder deren pharmazeutisch annehmbaren Salzen und CDP-Cholin oder dessen pharmazeutisch annehmbaren Salzen in der Form einer pharmazeutischen Zusammensetzung nach einem der Ansprüche 1-8 durchgeführt wird.

15. Synergetische Kombination von Harnsäure oder deren pharmazeutisch annehmbaren Salzen und CDP-Cholin oder dessen pharmazeutisch annehmbaren Salzen, zur Verwendung nach einem der vorhergehenden Ansprüche, **dadurch gekennzeichnet, dass** die genannte synergetische Kombination mittels parenteraler Verabreichung verabreicht wird.

16. Synergetische Kombination von Harnsäure oder deren pharmazeutisch annehmbaren Salzen und CDP-Cholin oder dessen pharmazeutisch annehmbaren Salzen, zur Verwendung nach einem der vorhergehenden Ansprüche, **dadurch gekennzeichnet, dass** die genannte synergetische Kombination intravenös verabreicht wird.

## Revendications

1. Composition pharmaceutique comprenant une quantité thérapeutiquement efficace d'acide urique ou ses sels pharmaceutiquement acceptables et de citicoline ou ses sels pharmaceutiquement acceptables pour le traitement neuroprotecteur chez des patients présentant un ictus.

2. Composition pharmaceutique selon la revendication 1, **caractérisée en ce que** la quantité thérapeutiquement efficace d'acide urique ou ses sels pharmaceutiquement acceptables est comprise entre 1 mg/ml et 4 mg/ml.

3. Composition pharmaceutique selon l'une quelconque des revendications 1-2, **caractérisée en ce que** la quantité thérapeutiquement efficace de citicoline ou ses sels pharmaceutiquement acceptables est comprise entre 2 mg/ml et 4 mg/ml.

4. Composition pharmaceutique selon l'une quelconque des revendications précédentes, comprenant un véhicule aqueux.

5. Composition pharmaceutique selon la revendication 4, **caractérisée en ce que** le véhicule aqueux est du sérum physiologique.

6. Composition pharmaceutique selon la revendication 5, **caractérisée en ce que** le sérum physiologique comprend du carbonate de lithium et du mannitol.

7. Composition pharmaceutique selon l'une quelconque des revendications précédentes, **caractérisée en ce que** ladite composition pharmaceutique est appliquée par administration parentérale.

8. Composition pharmaceutique selon l'une quelconque des revendications précédentes, **caractérisée en ce que** ladite composition pharmaceutique est administrée par voie intraveineuse.

9. Combinaison synergique d'acide urique ou ses sels pharmaceutiquement acceptables et de citicoline ou ses sels pharmaceutiquement acceptables pour une utilisation dans une thérapie combinée pour le traitement neuroprotecteur des patients présentant un ictus.

10. Combinaison synergique d'acide urique ou ses sels pharmaceutiquement acceptables et de citicoline ou ses sels pharmaceutiquement acceptables pour une utilisation selon la revendication 9, **caractérisée en ce que** la thérapie combinée est réalisée au moyen de l'administration simultanée ou séquentielle d'acide urique et de citicoline.

11. Combinaison synergique d'acide urique ou ses sels pharmaceutiquement acceptables et de citicoline ou ses sels pharmaceutiquement acceptables pour une utilisation selon l'une quelconque des revendications 9-10, **caractérisée en ce que** l'administration d'acide urique est réalisée dans une quantité comprise entre 1 mg/ml et 4 mg/ml dissoute dans du sérum physiologique avec du carbonate de lithium et du mannitol.

12. Combinaison synergique d'acide urique ou ses sels pharmaceutiquement acceptables et de citicoline ou ses sels pharmaceutiquement acceptables pour une utilisation selon l'une quelconque des revendications 9-11, **caractérisée en ce que** l'administration de citicoline est réalisée dans une quantité comprise entre 500 mg et 2000 mg.

13. Combinaison synergique d'acide urique ou ses sels pharmaceutiquement acceptables et de citicoline ou ses sels pharmaceutiquement acceptables pour une utilisation selon la revendication 9, **caractérisée en ce que** la thérapie combinée est réalisée au moyen de l'administration conjointe d'acide urique et de citicoline.

14. Combinaison synergique d'acide urique et de citicoline pour une utilisation selon la revendication 13, **caractérisée en ce que** l'administration conjointe d'acide urique ou ses sels pharmaceutiquement acceptables et de citicoline ou ses sels pharmaceutiquement acceptables est réalisée sous la forme d'une composition pharmaceutique selon l'une quelconque des revendications 1-8.

15. Combinaison synergique d'acide urique ou ses sels pharmaceutiquement acceptables et de citicoline ou ses sels pharmaceutiquement acceptables pour une utilisation selon l'une quelconque des revendications précédentes, **caractérisée en ce que** ladite combinaison synergique est administrée via une administration parentérale.

16. Combinaison synergique d'acide urique ou ses sels pharmaceutiquement acceptables et de citicoline ou ses sels pharmaceutiquement acceptables pour une utilisation selon l'une quelconque des revendications précédentes, **caractérisée en ce que** ladite combinaison synergique est administrée par voie intraveineuse.
